(19) Europäisches Patentamt
European Patent Office
Office européen des brevets

(11) Numéro de publication: **0 258 095 B1**

(12) # FASCICULE DE BREVET EUROPEEN

(45) Date de publication de fascicule du brevet: **12.06.91**

(51) Int. Cl.[5]: **C07D 215/40**, A61K 31/47, C07D 401/12, C07D 409/12

(21) Numéro de dépôt: **87401733.8**

(22) Date de dépôt: **24.07.87**

(54) **Nouveaux dérivés de la décahydroquinoléine, leur procédé de préparation et les nouveaux intermédiaires obtenus, leur application à titre de médicaments et les compositions pharmaceutiques les renfermant.**

(30) Priorité: **12.08.86 FR 8611620**
**09.07.87 FR 8709747**

(43) Date de publication de la demande:
**02.03.88 Bulletin 88/09**

(45) Mention de la délivrance du brevet:
**12.06.91 Bulletin 91/24**

(84) Etats contractants désignés:
**AT BE CH DE ES GB GR IT LI LU NL SE**

(56) Documents cités:
**EP-A- 0 068 699**

**JOURNAL OF MEDICINAL CHEMISTRY, vol. 25, no. 10, octobre 1982, pages 1125-1126, American Chemical Society; J. SZMUSZKOVICZ et al.: "Benzeneacetamide amines: structurally novel non-mmu opioids"**

**JOURNAL OF MEDICINAL CHEMISTRY, vol. 17, no. 11, novembre 1974, pages 1136-1139, American Chemical Society; I.W. MATHISON et al.: "Synthesis, stereochemistry, and anti-arrhythmic activity of some 8-substituted decahydroisoquinolines"**

(73) Titulaire: **ROUSSEL-UCLAF**
**35, boulevard des Invalides**
**F-75007 Paris(FR)**

(72) Inventeur: **Clemence, François**
**2, rue Turgot**
**F-75009 Paris(FR)**
Inventeur: **Le Martret, Odile**
**42, Avenue de Versailles**
**F-75016 Paris(FR)**
Inventeur: **Delevallée, Françoise**
**48-50, Avenue de La Dame Blanche**
**F-94120 Fontenay-Sous-Bois(FR)**
Inventeur: **Fortin, Michel**
**12, Passage Cottin**
**F-75018 Paris(FR)**

(74) Mandataire: **Bourgouin, André et al**
**Département des Brevets ROUSSEL UCLAF**
**B.P. no 9**
**F-93230 Romainville(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 258 095 B1

Rank Xerox (UK) Business Services

## Description

L'invention concerne de nouveaux dérivés de La décahydroquinoléine, leur procédé de préparation et les nouveaux intermédiaires obtenus, leur application à titre de médicaments et les compositions pharmaceutiques les renfermant.

L'invention a pour objet des composés de formule (I):

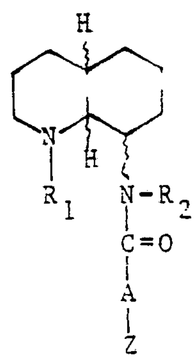

(I)

dans laquelle $R_1$ représente un radical alcoyle renformant de 1 à 5 atomes de carbone, $R_2$ représente un atome d'hydrogène ou un radical alcoyle renfermant de 1 à 5 atomes de carbone, A représente une chaîne $(CH_2)n$ dans laquelle n représente un nombre de 0 à 5, ou $-CH_2-O-$ ou A représente une chaîne alcoylène substituée par un radical alcoyle renfermant au total de 2 à 8 atomes de carbone, Z représente un radical phényle éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyl, nitro, amino, monoalkyl ou dialkylamino dont les radicaux alcoyles renferment de 1 à 5 atomes de carbone. un radical naphtyle, un radical indényle, un radical hétéromonocyclique renfermant 5 ou 6 chaînons, choisi parmi les radicaux thiazolyle, pyridinyle, oxazolyle, isoxazolyle, imidazolyle et thiényle, ou un radical hétérobicyclique choisi parmi les radicaux indolyle, quinolyle, benzo [b] thiényle, benzimidazolyle, benzoxazolyle et benzothiazolyle, tous ces radicaux étant éventuellement substitués par un ou plusieurs radicaux identiques ou différents choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes, étant entendu que lorsque Z représente un radical phényle substitué en ortho par un radical alcoxy, A ne peut pas représenter la valeur $(CH_2)n$ dans laquelle représente le nombre 0, lesdits composés de formule formule (I) pouvant être dans doutes les formes énantiomères et diastéréoisomères possibles et sous forme de sels d'addition avec les acides ou de sels d'ammonium quaternaire.

Par substituant alcoyle, alcoxy ou halogène, on entend de préférence méthyle, éthyle, propyle ou butyle linéaire ou ramifié, méthoxy, éthoxy, propoxy ou butoxy linéaire ou ramifié, fluoro, chloro, bromo ou iodo.

Dans les valeurs monoalkyl et dialkylamino, les radicaux alkyles sont préférentiellement les radicaux méthyle ou éthyle.

Lorsque A représente une chaîne $(CH_2)_{n'}$ n est de préférence égal à 0 ou 1.

Lorsque A représente une chaîne alcoylène substituée par un radical alcoyle, par alcoyle on entend de préférence méthyle ou éthyle et A est alors de préférence un radical 1,1-éthanediyl, 1-méthyl 1,2-éthanediyl, 1-méthyl ou 2-méthyl 1,3-propanediyl, 1-éthyl 1,2-éthanediyl.

Par ailleurs, un composé de formule (I) peut exister sous la forme de quatre racémates, ou paires d'énantiomères. Les énantiomères de chaque paire peuvent être séparés par des procédés classiques. L'invention couvre donc toutes les formes énantiomères et diastéréoisomères des composés de formule (I).

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléique, fumarique, succinique, tartrique, citrique, oxalique, glyoxylique, aspartique, alcanesulfoniques tels que l'acide méthane sulfonique et arylsulfoniques, tels que l'acide benzène sulfonique.

L'invention concerne aussi des composés de formule (I) sous forme de sels d'ammonium quaternaire.

Par sels d'ammonium quaternaire, on entend les composés de formule (I) quaternisés par des produits de type R-Y, R étant un radical alcoyle ayant de 1 à 4 atomes de carbone tel qu'un radical méthyle, éthyle, n-propyle ou isopropyle et Y un anion halogénure, par exemple, un chlorure, un bromure, un iodure.

L'invention concerne notamment les produits de formule (I) dans laquelle $R_1$ représente un radical alcoyle renfermant de 1 à 5 atomes de carbone, A représente une chaîne alkylène linéaire $-(CH_2)_n-$, n représentant un nombre entier pouvant varier entre 0 et 5 ou une chaîne alkylène ramifiée renfermant de 2 à 8 atomes de carbone, $R_2$ représente un atome d'hydrogène ou un radical alcoyle renfermant de 1 à 5 atomes de carbone, Z représente un radical phényle substitué par X et X', identiques ou différents, représentant un atome d'hydrogène, un radical alcoyle renfermant de 1 à 4 atomes de carbone, un radical alkoxy renfermant de 1 à 4 atomes de carbone, un radical hydroxyle, un atome d'halogène, un radical trifluorométhyle, nitro, amino, monoalkylamino ou dialkylamino, étant entendu que lorsque X ou X' représente un radical alkoxy en position ortho du noyau phényl, n ne peut représenter la valeur O lorsque A représente $-(CH_2)_n-$, lesdits composés de formule (I) pouvant être dans toutes les formes énantiomères et diastéréoisomères possibles et sous forme de sels d'addition avec les acides ou de sels d'ammonium quaternaire.

L'invention concerne notamment les composés de formule (I) caractérisés en ce que la jonction des cycles est cis ainsi que leurs sels d'addition avec les acides et ceux caractérisés en ce que A représente un groupe $-CH_2$ ou $-CH_2-O-$, ainsi que leurs sels d'addition avec les acides.

L'invention a particulièrement pour objet les composés de formule (I) caractérisés en ce que $R_1$ représente un radical méthyle ou éthyle, $R_2$ représente un atome d'hydrogène, un radical méthyle ou éthyle, Z représente un radical phényle substitué éventuellement par un ou deux radicaux choisis parmi les radicaux méthyle ou éthyle, méthoxy ou éthoxy, les atomes de chlore ou de brome, les radicaux trifluorométhyle ou nitro ainsi que leurs sels d'addition avec les acides et ceux caractérisés en ce que $R_1$ représente un radical méthyle ou éthyle, $R_2$ représente un atome d'hydrogène, un radical méthyle ou éthyle, Z représente un radical naphtyle, pyridinyle, benzo [b] thiényle, ainsi que leurs sels d'addition avec les acides.

L'invention a tout particulièrement pour objet le (4a alpha, 8 alpha, 8a alpha) (±) N-(décahydro 1-méthyl 8-quinoléinyl) 3,4-dichloro N-méthyl phényl acétamide et ses sels d'addition avec acides et le (4a alpha, 8 alpha, 8a alpha) (±) N-(décahydro 1-méthyl 8-quinoléinyl) 2-(3,4-dichlorophénoxy) N-méthyl acétamide et ses sels d'addition avec les acides.

L'invention concerne aussi un procédé de préparation des produits de formule (II), caractérisé en ce que l'on soumet la 8-chloro 5,6,7,8-tétrahydroquinoléine, à l'action d'un produit de formule $X-R_1$, $R_1$ étant un radical alcoyle renfermant de 1 à 5 atomes de carbone et X représentant un atome d'halogène pour obtenir un produit de formule (II):

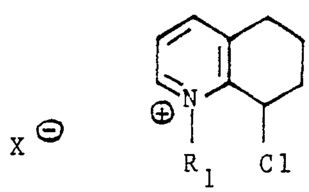

(II)

que l'on fait réagir avec une amine de formule $NH_2-R_2$, $R_2$ étant un atome d'hydrogène ou un radical alcoyle renfermant de 1 à 5 atomes de carbone, pour obtenir un produit de formule (III):

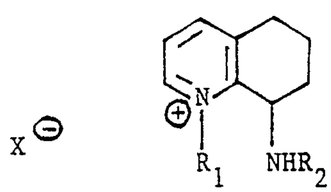

(III)

que l'on réduit pour obtenir un produit de formule (IV) sous toutes les formes énantiomères et diastéréoisomères possibles:

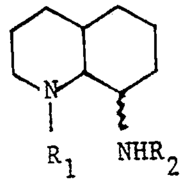

(IV)

que l'on condense avec un acide de formule (V) ou un dérivé fonctionnel de celui-ci:

HOOC-(A)-z (v)

A et Z ayant toutes les significations données précédemment, pour obtenir un produit de formule (I) dans toutes les formes énantiomèrs et diastéréoisoméres possibles, pouvant être dédoublé pour obtenir les formes optiquement actives et que l'on traite si désiré avec un acide minéral ou organique pour en former le sel.

Dans un mode de réalisation préféré du procédé de l'invention:

- Le dérivé halogéné X-$R_1$ est un iodure d'alcoyle et la réaction s'effectue dans l'acétonitrile.
- La réduction du produit de formule (III) est effectuée par hydrogénation catalytique. Le catalyseur utilisé est de préférence l'oxyde de platine.
- L'activation de la fonction carboxyle du composé de formule (V) pour réaliser la condensation avec le composé de formule (IV) s'effectue en présence de carbonyldiimidazole ou de dicyclohexylcarbodiimide. On peut également activer l'acide de formule (V) sous la forme d'un chlorure d'acide ou d'anhydride mixte.

Par ailleurs, les 2 isomères de formule (IV) correspondant aux orientations alpha ou béta du groupement

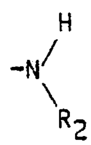

par rapport au cycle sont séparés par chromatographie. Chacun des racémiques obtenus peut être dédoublé par des méthodes usuelles par exemple par séparation des sels des diastéréoisomères obtenus à partir d'acides optiquement actifs.

Les composés de formule (I) tels que définis ci-dessus ainsi que leurs sels d'addition avec les acides présentent d'intéressantes propriétés pharmacologiques. Ils présentent en particulier une forte affinité pour les récepteurs opiacés et notamment pour les récepteurs K et sont doués de propriétés analgésiques centrales.

Ils sont doués également de propriétés diurétiques, de propriétés anti-arythmiques, anti-ischémiques cérébrales et hypotensives.

Ces propriétés justifient leur application en thérapeutique et l'invention a également pour objet à titre de médicaments, les produits tels que définis par la formule (I) ci-dessus ainsi que par leurs sels d'addition avec les acides pharmaceutiquement acceptables et leurs sels d'ammonium quaternaire.

Les médicaments, objet de l'invention, permettent notamment de soulager une douleur quelle qu'en soit l'origine, par exemple une douleur de nature musculaire, articulaire ou nerveuse.

Ils peuvent être aussi utilisés dans le traitement des douleurs dentaires, des migraines, du zona, dans le traitement des douleurs intenses, en particulier rebelles aux antalgiques périphériques, par exemple au cours du processus néoplasique, dans le traitement des pancréatites, coliques néphrétiques ou biliaires, dans le traitement des douleurs post-opératoires et post-traumatiques.

La posologie varie notamment en fonction de la voie d'administration, de l'affection traitée et du sujet en cause.

Par exemple, chez l'adulte, elle peut varier entre 20 et 400 mg de principe actif par jour, par voie orale et entre 5 et 100 mg par jour, par voie parentérale.

Les médicaments, objet de l'invention, trouvent aussi leur emploi dans le traitement des arythmies.

La dose usuelle, dans ce traitement, variable selon le dérivé utilisé, le sujet et l'affection en cause, peut être par exemple de 50 mg à 1 g par jour.

Par voie orale, le principe actif peut être administré à la dose quotidienne de 200 mg à 800 mg, par

exemple pour le traitement des arythmies ventriculaires, supraventriculaires et jonctionnelles, soit environ de 3 mg à 12 mg par kilogramme de poids corporel.

Les médicaments, objet de l'invention peuvent aussi être utilisés dans le traitement des syndromes oedémateux, de l'insuffisance cardiaque, de certaines obésités, des cirrhoses, dans le traitement des oedèmes sévères et réfractaires, en particulier ceux de l'insuffisance cardiaque congestive et dans le traitement au long cours de l'hypertention artérielle.

La dose quotidienne de principe actif est variable. Elle peut être par exemple de 60 à 100 mg/jour par voie orale.

L'invention s'étend aux compositions pharmaceutiques renfermant comme principe actif les médicament définis ci-dessus.

Ces compositions pharmaceutiques peuvent être adminitrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 et 400 mg de principe actif par jour, par voie orale et entre 5 et 100 mg par jour, par voie parentérale.

Par ailleurs, les composés de formule (III) et (IV) sont des produits chimiques nouveaux.

L'invention a donc pour objet ces produits à titre de produits industriels nouveaux, notamment à titre de produits intermédiaires nécessaires à la mise en oeuvre du procédé.

La 8-chloro 5,6,7,8-tétrahydroquinoléine, utilisée comme produit de départ dans le procédé de l'invention, est préparée par chloruration de la 5,6,7,8-tétrahydroquinoléine N-oxyde selon la méthode indiquée dans le brevet US 3 991 065.

Les exemples donnés ci-après illustrent l'invention sans toutefois la limiter.

**Exemple 1 : (4a alpha, 8 alpha, 8a alpha) (±) N-(décahydro 1-méthyl 8-quinoléinyl) 3,4-dichloro N-méthyl phényl acétamide et son chlorhydrate.**

**Stade A :** Iodure de 8-chloro 1-méthyl 5,6,7,8-tétrahydro 1-quinolinium

On agite 68 heures à température ambiante une solution renfermant 13,70 g de 5,6,7,8-tétrahydro 8-chloroquinoléine (préparation donnée ci-dessous), 86 cm3 d'acétonitrile et 15,2 cm3 d'iodure de méthyle. On amorce la cristallisation après 50 minutes de réaction, essore, rince à l'acétonitrile puis à l'éther, sèche sous pression réduite à 20¤C et obtient 29,96 g de produit attendu. F = 175¤C.

Préparation de la 5,6,7,8-tétrahydro 8-chloroquinoléine utilisée comme produit de départ.

On ajoute lentement à température ambiante 3 cm3 de chlorure de méthane sulfonyle à 1,49 g de 5,6,7,8-tétrahydroquinoléine N-oxyde sous agitation et atmosphère inerte. On chauffe ensuite 4 heures à 80-82¤C puis refroidit à 20¤C. On verse sur 20 cm3 de solution saturée de bicarbonate de sodium puis ajoute lentement jusqu'à obtention d'un pH alcalin du bicarbonate de sodium. On extrait par du chlorure de méthylène, lave à l'eau, sèche les solutions organiques réunies, distille à sec sous pression réduite et obtient 1,53 g de produit attendu sous forme d'une huile.

**Stade B :** Iodure de 1-méthyl 8-(méthylamino) 5,6,7,8-tétrahydro 1-quinolinium.

On introduit 8,02 g de produit obtenu au stade A dans 40 cm3 de tétrahydrofuranne et ajoute ensuite en une fois à la pipette 7,4 cm3 d'une solution éthanolique de monoéthylamine. On agite 20 heures à température ambiante la suspension obtenue, essore les cristaux, rince par du tétrahydrofuranne et de l'éther, sèche sous pression réduite à 20¤C et obtient 4,76 g de produit attendu. F = 180¤C.

**Stade C :** (4a alpha, 8 alpha, 8a alpha) (±) décahydro N,1-diméthyl 8-quinoléinamine et (4a alpha, 8 beta ,

8a alpha) (+) décahydro N,1-diméthyl 8-quinoléinamine.

On ajoute en une fois 1,029 g d'oxyde de platine dans une solution de 10,29 g de produit obtenu au stade B dans 150 cm3 d'acide acétique. On hydrogène la suspension à 22-24¤C durant 50 heures. Après 29 heures d'hydrogénation, on a rajouté 1 g d'oxyde de platine. On ajoute en fin de réaction 30 cm3 d'eau, filtre, rince au méthanol, distille sous pression réduite. On reprend le résidu dans 200 cm3 d'éther, ajoute lentement sous agitation et en refroidissant 200 cm3 de lessive de soude diluée au demi. On agite, décante, extrait la phase aqueuse à l'éther, sèche les phases organiques et distille à sec sous pression réduite. On obtient 5,60 g de produit qui est un mélange des 2 diastéréoisomères à jonction de cycle cis.

Séparation des 2 diastéréoisomères par chromatographie préparative.

On effectue une chromatographie préparative sur silice (éluant : acétate d'éthyle-méthanol-triéthylamine 85-10-5) à pression atmosphérique. On laisse en contact 1 heure avec l'éluant et chromatographie 5,5 g de base brute. On récupère en tête une fraction de 579 mg de mélange de 3 produits mobiles et amène ensuite à sec sous pression réduite les fractions correspondant à l'isomère ayant l'orientation 8 alpha. On obtient 1,122 g de produit à orientation 8 alpha. On amène à sec sous pression réduite les fractions correspondant à l'isomère ayant l'orientation 8 béta. On obtient 3,361 g de produit à orientation 8 béta.

**Stade D :** (4a alpha, 8 alpha, 8a alpha) (+) N-(décahydro 1-méthyl 8-quinoléinyl) 3,4-dichloro N-méthyl phényl acétamide et son chlorhydrate.

On mélange à 20¤C pendant 1 heure 2,13 g d'acide 3,4-dichlorophényl acétique, 1,69 g de carbonyldiimidazole et 20 cm3 de tètrahydrofuranne. On ajoute ensuite 1,46 g de diastéréoisomère 8 alpha obtenu comme au stade C en solution dans 5 cm3 de tétrahydrofuranne et agite 3 heures 30 minutes à 20-22¤C. On élimine le tétrahydrofuranne sous pression réduite, reprend le résidu par 50 cm3 d'éther, lave par une solution saturée de bicarbonate de sodium, puis par de l'eau salée, sèche, rince et concentre sous pression réduite. On obtient 3,73 g de produit brut que l'on purifie par passage à l'oxalate. On dissout 3,645 g de produit brut dans 10 cm3 d'éthanol et ajoute 1,5 g d'acide oxalique en solution dans 2,5 cm3 d'éthanol. On filtre l'insoluble obtenu, rince à l'éthanol, sèche sous pression réduite à 20¤C et obtient 464 mg de produit. On ajoute lentement aux liqueurs mères 120 cm3 d'éther, une gomme précipite. On décante la solution surnageante, rince à l'éther, reprend la gomme à l'eau et alcalinise par du bicarbonate de sodium en présence de 50 cm3 d'éther. On décante, lave à l'eau salée, sèche, concentre à sec sous pression réduite. On obtient 2,29 g de produit attendu sous forme d'un extrait sec huileux qui cristallise. F = 92¤C.

Préparation du chlorhydrate.

On dissout 2,15 g d'extrait sec dans 5 cm3 d'éthanol à 50-60¤C, filtre, rince à l'éthanol et à l'éther. On ajoute 2 cm3 d'acide chlorhydrique dans l'éthanol, amorce la cristallisation, obtient une prise en masse épaisse, dilue par de l'éther, essore, rince à l'éthanol avec un mélange éther-éthanol (3-2) puis à l'éther. On sèche sous pression réduite à 70¤C et obtient 2,05 g de produit attendu. F = 253¤C.

**Exemple 2 : (4a alpha, 8 beta, 8a alpha) (+) N-(décahydro 1-méthyl 8-quinoléinyl) 3,4-dichloro N-méthyl phényl acétamide et son chlorhydrate.**

On introduit 1,46 g de dicyclohexylcarbodiimide dans une solution renfermant 1,46 g d'acide 3,4-dichlorophénylacétique, 1,122 g de diastéréoisomère 8 béta obtenu au stade C de l'exemple 1 et 20 cm3 de chlorure de méthylène. On agite 3 heures 30 minutes, filtre, rince au chlorure de méthylène, concentre à sec le filtrat sous pression réduite et reprend le résidu à l'éther, lave avec une solution aqueuse saturée en bicarbonate de sodium puis à l'eau salée, sèche, concentre à sec sous pression réduite. On obtient 2,845 g de produit que l'on reprend par 15 cm3 d'éther, amorce la cristallisation, essore, rince à l'éther, sèche sous pression réduite et obtient 2,053 g de produit. On purifie 1,885 g de produit par chromatographie sur silice (éluant : acétate d'éthyle à 2% de triéthylamine). Après avoir amené à sec les fractions homogènes, on récupère 1,477 g de produit que l'on dissout dans du chlorure de méthylène et filtre. On distille le chlorure de méthylène sous pression réduite tout en introduisant de l'éther isopropylique. Le produit cristallise en cours de distillation ; on essore, rince à l'éther isopropylique, sèche sous pression réduite à 50¤C et obtient 1,27 g de produit attendu. F = 139¤C sous forme de base.

Préparation du chlorhydrate.

On dissout 1,2 g de produit obtenu ci-dessus dans 4,8 cm3 d'éthanol et 1 cm3 d'éthanol renfermant de l'acide chlorhydrique (5,75N) à 20¤C, filtre, rince à l'éthanol, concentre à sec le filtrat sous pression réduite. On triture le résidu dans 10 cm3 d'éther, essore, rince à l'éther, sèche sous pression réduite à 70¤C. On obtient 1,259 g de produit attendu. F≃ 258¤C.

En opérant comme à l'exemple 1 au départ de la (4a alpha, 8 alpha, 8a alpha) (±) décahydro N,1-diméthyl 8-quinoléinamine et de l'acide convenable, on a préparé les produits des exemples 3 à 11 dont les noms suivent.

**Exemple 3 : [4a alpha, 8 alpha, 8a alpha] (±) N-(décahydro 1-méthyl 8-quinoléinyl) N-méthyl 4-nitrophényl acétamide et son chlorhydrate.**
**Exemple 4 : [4a alpha, 8 alpha, 8a alpha] (±) N-(décahydro 1-méthyl 8-quinoléinyl) N-méthyl 4-(trifluorométhyl) phényl acétamide et son E-butène dioate.**
**Exemple 5 : [4a alpha, 8 alpha, 8a alpha] 4-bromo N-(décahydro 1-méthyl 8-quinoléinyl) N-méthyl phényl acétamide et son chlorhydrate.**
**Exemple 6 : [4a alpha, 8 alpha, 8a alpha] (±) N-(décahydro 1-méthyl 8-quinoléinyl) 4,N-diméthyl phényl acétamide et son chlorhydrate.**
**Exemple 7 : [4a alpha, 8 alpha, 8a alpha] (±) N-(décahydro 1-méthyl 8-quinoléinyl) 2-(3,4-dichlorophénoxy) N-méthyl acétamide et son E-butène dioate.**
**Exemple 8 : [4a alpha, 8 alpha, 8a alpha] (±) N-(décahydro 1-méthyl 8-quinoléinyl) 3,4-diméthoxy N-méthyl phényl acétamide et son E-butène dioate.**
**Exemple 9 : [4a alpha, 8 alpha, 8a alpha] (±) N-(décahydro 1-méthyl 8-quinoléinyl) N-méthyl 1-naphtyl acétamide et son E-butène dioate.**
**Exemple 10 : [4a alpha, 8 alpha, 8a alpha] (±) N-(décahydro 1-méthyl 8-quinoléinyl) N-méthyl 4-pyridyl acétamide et son oxalate.**
**Exemple 11 : [4a alpha, 8 alpha, 8a alpha] (±) N-(décahydro 1-méthyl 8-quinoléinyl) N-méthyl 4-benzo (b) thiényl acétamide.**

L'acide utilisé, les résultats de la microanalyse et les points de fusion des produits obtenus figurent dans le tableau I ci-après.

TABLEAU I

| Exemple | Acide utilisé au départ | Microanalyse : Calculé/Trouvé C% | H% | N% | Cl% | Br% | S% | F (°C) |
|---|---|---|---|---|---|---|---|---|
| 3 | Acide p-nitrophényl acétique | 59,75<br>59,6 | 7,39<br>7,3 | 11,00<br>10,8 | 9,28<br>9,4 | | | # 228<br>(décomp) |
| 4 | Acide p-trifluorométhyl phényl acétique | 59,49<br>59,7 | 6,45<br>6,77 | 5,78<br>5,7 | 11,76<br>11,45 | | | 180 |
| 5 | Acide p-bromophényl acétique | 54,88<br>54,6 | 6,79<br>6,9 | 6,74<br>6,5 | 8,52<br>8,2 | 19,22<br>19,0 | | > 260 |
| 6 | Acide p-tolyl acétique | 68,45<br>68,4 | 8,90<br>9,2 | 7,98<br>7,8 | 10,10<br>10,3 | | | > 260 |
| 7 | Acide 3,4-dichlorophénoxy acétique | 55,09<br>55,1 | 6,03<br>6,1 | 5,59<br>5,5 | 14,14<br>14,0 | | | 205 |
| 8 | Acide 3,4-diméthoxyphényl acétique | 63,01<br>63,1 | 7,61<br>7,7 | 5,88<br>5,6 | | | | # 150<br>(décomp) |
| 9 | Acide 1-naphtyl acétique | 69,50<br>69,6 | 7,34<br>7,4 | 6,00<br>6,0 | | | | # 199<br>(décomp) |
| 10 | Acide 4-pyridyl acétique | 54,88<br>54,6 | 6,49<br>6,4 | 8,73<br>8,7 | | | | 187 |
| 11 | Acide 4-thianaphtène acétique | 64,18<br>63,9 | 7,44<br>7,4 | 7,13<br>7,2 | 9,02<br>9,3 | | 8,16<br>8,0 | > 260 |

**Exemple 12 :**

On a préparé des comprimés répondant à la formule suivante :

- produit de l'exemple 1 .............................................. 200 mg
- excipient q.s.p.................................................... 800mg.

(détail de l'excipient : lactose, talc, amidon, stéarate de magnésium).

Exemple 13 :

On a préparé un soluté injectable (voie intra-musculaire) répondant à la formule suivante :

- produit de l'exemple 7 .............................................. 50 mg
- solvant stérile q.s.p.............................................. 5 cm3

**ETUDE PHARMACOLOGIQUE**

1) Liaison au récepteur opiacé K in vitro.

On utilise des culots membranaires conservés à -30¤C (éventuellement pendant environ 30 jours) et préparés à partir de cervelets de cobayes.

Ces culots sont remis en suspension dans le tampon Tris pH 7,7. On répartit les fractions de 2 ml dans des tubes à hémolyse et ajoute de la $9^3H$ éthylkétocyclazocine 1nM et le produit à étudier. Le produit est d'abord testé à $5X10^{-6}M$ (en triple). Lorsque le produit testé déplace de plus de 50% la radioactivité liée spécifiquement au récepteur, il est testé à nouveau selon une gamme de 7 doses afin de déterminer la dose qui inhibe de 50% la radioactivité liée spécifiquement au récepteur. On détermine ainsi la concentration inhibitrice 50%.

La liaison non spécifique est déterminée par addition de produit connu sous le nom U-50488 H (Lahti et al. 1982, Life Sci. 31, 2257) à $10^{-5}M$ (en triple). On incube à 25¤C pendant 40 minutes, remet au bain-marie à 0¤C, 5 minutes, filtre sous vide, rince au tampon Tris pH 7,7 et compte la radioactivité en présence du scintillant Triton®.

Le résultat est exprimé :

- directement en concentration inhibitrice 50% ($CI_{50}$), c'est-à-dire en concentration de produit étudié, exprimée en nM, nécessaire pour déplacer 50% de la radioactivité spécifique fixée sur le récepteur étudié.

Résultats :

La $CI_{50}$ trouvée est de 5,6 nanomoles pour le produit de l'exemple 1 et de 5,3 nanomoles pour le produit de l'exemple 7.

2) Action antiarythmique chez le rat.

On trachéotomise des rats mâles pesant 300-350 g anesthésiés par voie intrapéritonéale à l'aide de 1,20g/kg d'uréthane et les soumet à une respiration artificielle (40-50 insufflations de 3 ml/minute).

On implante des aiguilles en sous cutané de manière à enregistrer L'électrocardiogramme des rats sur le signal en dérivation DII.

On administre les produits à tester par voie intraveineuse.

Cinq minutes après l'administration du produit, on perfuse la veine jugulaire des rats avec 10 µg/mn sous 0,2 ml d'une solution d'aconitine et on note le temps d'apparition des troubles du rythme cardiaque.

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que certains des produits de la présente demande sont doués de bonnes propriétés antiarythmiques.

| Produit de l'exemple 2 | Dose mg/kg | Pourcentage d'allongement du temps |
|---|---|---|
| | 10 | + 43 % |
| | 5 | + 28 % |
| | 2,5 | + 16 % |

**Revendications**

**1.** Composés de formule (I) :

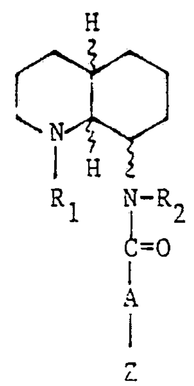

(I)

dans laquelle $R_1$ représente un radical alcoyle renfermant de 1 à 5 atomes de carbone, $R_2$ représente un atome d'hydrogène ou un radical alcoyle renfermant de 1 à 5 atomes de carbone, A représente une chaîne $(CH_2)_n$ dans laquelle n représente un nombre de 0 à 5, ou $-CH_2-O-$ ou A représente une chaîne alcoylène substituée par un radical alcoyle renfermant au total de 2 à 8 atomes de carbone, Z représente un radical phényle éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone. un radical naphtyle, un radical indényle, un radical hétéromonocyclique renfermant 5 ou 6 chaînons, choisi parmi les radicaux thiazolyle, pyridinyle, oxazolyle, isoxazolyle, imidazolyle et thiényle, ou un radical hétérobicyclique choisi parmi les radicaux indolyle, quinolyle, benzo [b] thiényle, benzimidazolyle, benzoxazolyle et benzothiazolyle, tous ces radicaux étant éventuellement substitués par un ou plusieurs radicaux identiques ou différents choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes, étant entendu que lorsque z

représente un radical phényle substitué en ortho par un radical alcoxy, A ne peut pas représenter la valeur $(CH_2)_n$ dans laquelle n représente le nombre 0, lesdits composés de formule formule (I) pouvant être dans toutes les formes énantiomères et diastéréoisomères possibles et sous forme de sels d'addition avec les acides ou de sels d'ammonium quaternaire.

**2.** Composés de formule (I), tels que définis à la revendication 1, caractérisés en ce que la jonction des cycles est cis ainsi que leurs sels d'addition avec les acides.

**3.** Composés de formule (I), tels que définis à la revendication 1 et 2, caractérisés en ce que A représente un groupe $-CH_2-$ ou $-CH_2-0-$, ainsi que leurs sels d'addition avec les acides.

**4.** Composés de formule (I), tels que définis aux revendications 1 à 3, caractérisés en ce que $R_1$ représente un radical méthyle ou éthyle, $R_2$ représente un atome d'hydrogène, un radical méthyle ou éthyle, Z représente un radical phényle substitué éventuellement par un ou deux radicaux choisis parmi les radicaux méthyle ou éthyle, méthoxy ou éthoxy, les atomes de chlore ou de brome, les radicaux trifluorométhyle ou nitro ainsi que leurs sels d'addition avec les acides.

**5.** Composés de formule (I), tels que définis aux revendications 1 à 3, caractérisés en ce que $R_1$ représente un radical méthyle ou éthyle, $R_2$ représente un atome d'hydrogène, un radical méthyle ou éthyle, Z représente un radical naphtyle, pyridinyle, benzo [b] thiényle, ainsi que leurs sels d'addition avec les acides.

**6.** Le (4a alpha, 8 alpha, 8a alpha) $(\pm)$ N-(décahydro 1-méthyl 8-quinoléinyl) 3,4-dichloro N-méthyl phényl acétamide et ses sels d'addition avec acides et le (4a alpha, 8 alpha, 8a alpha) $(\pm)$ N-(décahydro 1-méthyl 8-quinoléinyl) 2-(3,4-dichlorophénoxy) N-méthyl acétamide et ses sels d'addition avec les acides.

**7.** Procédé de préparation des produits de formule (I), caractérisé en ce que l'on soumet la 8-chloro 5,6,7,8-tétrahydroquinoléine, à l'action d'un produit de formule $X-R_1$, $R_1$ étant un radical alcoyle renfermant de 1 à 5 atomes de carbone et X représentant un atome d'halogène pour obtenir un produit de formule (II) :

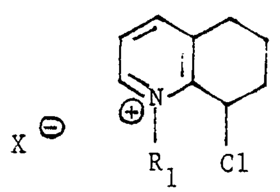

(II)

que l'on fait réagir avec une amine de formule $NH_2-R_2$, $R_2$ étant un atome d'hydrogène ou un radical alcoyle renfermant de 1 à 5 atomes de carbone, pour obtenir un produit de formule (III) :

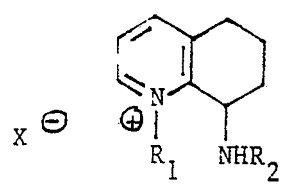

(III)

que l'on réduit pour obtenir un produit de formule (IV) sous toutes les formes énantiomères et diastéréoisomères possibles :

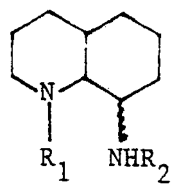

(IV)

que l'on condense avec un acide de formule (V) ou un dérivé fonctionnel de celui-ci :

HOOC-(A)-Z (V)

A et Z ayant toutes les significations données précédemment, pour obtenir un produit de formule (I) dans toutes les formes énantiomères et diastéréoisomères possibles, pouvant être dédoublé pour obtenir les formes optiquement actives et que l'on traite si désiré avec un acide minéral ou organique pour en former le sel.

**8.** A titre de médicaments, les produits de formule (I), tels que définis aux revendications 1 à 5.

**9.** A titre de médicaments, les produits de formule (I), tels que définis à la revendication 6.

**10.** Compositions pharmaceutiques contenant, à titre de principe actif, un médicament selon l'une des revendications 8 ou 9.

**11.** A titre de produits intermédiaires, les produits de formule (III) et (IV).

## Claims

**1.** Compounds of formula (I):

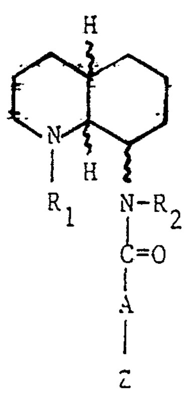

(I)

in which $R_1$ represents an alkyl radical containing 1 to 5 carbon atoms, $R_2$ represents a hydrogen atom or an alkyl radical containing 1 to 5 carbon atoms, A represents a $(CH_2)_n$ chain in which n represents a number from 0 to 5, or $-CH_2-O-$, or A represents an alkylene chain substituted by an alkyl radical containing in total 2 to 8 carbon atoms, Z represents a phenyl radical optionally substituted by one or more radicals, identical or different, chosen from the group constituted by alkyl radicals containing 1 to 5 carbon atoms, alkoxy radicals containing 1 to 5 carbon atoms, halogen atoms, hydroxyl, trifluoromethyl, nitro or amino radicals, or monoalkyl- or dialkylamino radicals, of which the alkyl radicals contain 1 to 5 carbon atoms, a naphthyl radical, an indenyl radical, a heteromonocyclic radical containing 5 or 6 links, chosen from thiazolyl, pyridinyl, oxazolyl, isoxazolyl, imidazolyl and thienyl radicals, or a heterobicyclic radical chosen from indolyl, quinolyl, benzo-[b]-thienyl, benzimidazolyl, benzoxazolyl and benzothiazolyl radicals, all these radicals being optionally substituted by one or more radicals, identical or different, chosen from the group constituted by alkyl radicals containing 1 to 5 carbon atoms, alkoxy radicals containing 1 to 5 carbon atoms, trifluoromethyl, nitro and amino radicals and monoalkyl- or dialkylamino radicals of which the alkyl radicals contain 1 to 5 carbon atoms, and

phenyl radicals optionally substituted by one or more alkyl radicals containing 1 to 5 carbon atoms, alkoxy radicals containing 1 to 5 carbon atoms or halogens, it being understood that when Z represents a phenyl radical substituted in ortho position by an alkoxy radical, A cannot represent the value $(CH_2)_n$ in which n represents the number 0, the said compounds of formula (I) being able to be in all the possible enantiomeric and diastereoisomeric forms and in the form of addition salts with acids or of quaternary ammonium salts.

**2.** Compounds of formula (I) as defined in claim 1, characterized in that the junction of the rings is cis, as well as their addition salts with acids.

**3.** Compounds of formula (I), as defined in claim 1 and 2, characterized in that A represents a $-CH_2$ or $-CH_2-O-$ group, as well as their addition salts with acids.

**4.** Compounds of formula (I), as defined in claims 1 to 3, characterized in that $R_1$ represents a methyl or ethyl radical, $R_2$ represents a hydrogen atom, a methyl or ethyl radical, Z represents a phenyl radical optionally substituted by one or two radicals chosen from methyl or ethyl, methoxy or ethoxy radicals, chlorine or bromine atoms, trifluoromethyl or nitro radicals, as well as their addition salts with acids.

**5.** Compounds of formula (I), as defined in claims 1 to 3, characterized in that $R_1$ represents a methyl or ethyl radical, $R_2$ represents a hydrogen atom, a methyl or ethyl radical, Z represents a naphthyl, pyridinyl or benzo-[b]-thienyl radical, as well as their addition salts with acids.

**6.** [-(4a-alpha, 8-alpha, 8a-alpha)] (±) N-(decahydro-1-methyl-8-quinolinyl)-3,4-dichloro-N-methyl phenyl acetamide and its addition salts with acids and (4a-alpha, 8-alpha, 8a-alpha) (±) N-(decahydro-1-methyl-8-quinolinyl) [2-(3,4-dichlorophenoxy)]-N-methyl acetamide and its addition salts with acids.

**7.** Preparation process for products of formula (I), characterized in that 8-chloro-5,6,7,8-tetrahydroquinoline is subjected to the action of a product of formula $X-R_1$, $R_1$ being an alkyl radical containing 1 to 5 carbon atoms and X representing a halogen atom, in order to obtain a product of formula (II):

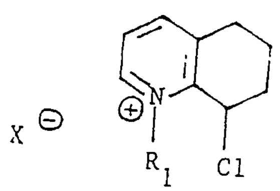

(II)

which is reacted with an amine of formula $NH_2-R_2$, $R_2$ being a hydrogen atom or an alkyl radical containing 1 to 5 carbon atoms, in order to obtain a product of formula (III):

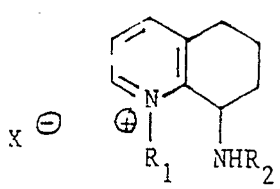

(III)

which is reduced to obtain a product of formula (IV) in all the possible enantiomeric and diastereoisomeric forms:

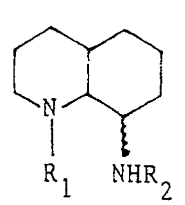

(IV)

which is condensed with an acid of formula (V) or a functional derivative of this acid:

HOOC-(A)-Z (V)

A and Z having all the meanings given previously, in order to obtain a product of formula (I) in all the possible enantiomeric and diastereoisomeric forms, able to be resolved in order to obtain the optically active forms and which is treated, if desired, with a mineral or organic acid to form the salt.

**8.** As medicaments, the products of formula (I), as defined in claims 1 to 5.

**9.** As medicaments, the products of formula (I), as defined in claim 6.

**10.** Pharmaceutical compositions containing, as active ingredient, a medicament according to one of claims 8 or 9.

**11.** As intermediate products, the products of formula (III) and (IV).

## Ansprüche

**1.** Verbindungen der Formel (I)

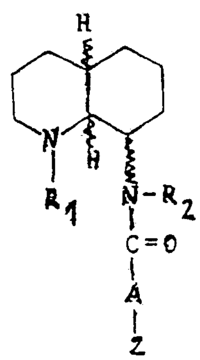

(I)

worin $R_1$ einen Alkylrest mit 1 bis 5 Kohlenstoffatomen bedeutet, $R_2$ ein Wasserstoffatom oder einen Alkylrest mit 1 bis 5 Kohlenstoffatomen darstellt, A eine Kette $(CH_2)_n$, worin n für eine Zahl von 0 bis 5 steht, oder $-CH_2-O-$ bedeutet oder A eine Alkylenkette, substituiert durch einen Alkylrest mit insgesamt 2 bis 8 Kohlenstoffatomen darstellt, Z einen Phenylrest wiedergibt, der gegebenenfalls durch einen oder mehrere identische oder verschiedene Reste substituiert ist, ausgewählt unter der Gruppe, bestehend aus den Alkylresten mit 1 bis 5 Kohlenstoffatomen, Alkoxyresten mit 1 bis 5 Kohlenstoffatomen, Halogenatomen, Hydroxyl-, Trifluormethyl-, Nitro-, Amino-, Monoalkyl- oder Dialkylaminoresten, deren Alkylreste 1 bis 5 Kohlenstoffatome enthalten, einem Naphthylrest, einem Indenylrest, einem heteromonocyclischen Rest mit 5 oder 6 Kettengliedern, ausgewählt unter den Resten Thiazolyl, Pyridinyl, Oxazolyl, Isoxazolyl, Imidazolyl und Thienyl, oder einem heterobicyclischen Rest, ausgewählt unter den Resten Indolyl, Chinolyl, Benzo[b]thienyl, Benzimidazolyl, Benzoxazolyl und Benzothiazolyl, wobei alle diese Reste gegebenenfalls substituiert sind durch einen oder mehrere identische oder verschiedene Reste, ausgewählt aus der Gruppe,bestehend aus den Alkylresten mit 1 bis 5 Kohlenstoffatomen, Alkoxyresten mit 1 bis 5 Kohlenstoffatomen, den Trifluormethyl-, Nitro-, Amino-, Monoalkyl- oder Dialkylaminoresten, deren Alkylreste 1 bis 5 Kohlenstoffatome enthalten, und Phenyl, gegebenenfalls substituiert durch einen oder mehrere Alkylreste mit 1 bis 5 Kohlenstoffatomen, Alkoxy mit 1 bis 5 Kohlenstoffatomen oder Halogene, wobei, falls Z einen in ortho-Stellung durch einen Alkoxyrest substituierten Phenylrest bedeutet, A nicht den Wert $(CH_2)_n$ mit $n = 0$ haben kann, wobei diese Verbindungen der Formel (I) in allen möglichen enantiomeren und diastereoisomeren Formen und in Form von Additionssalzen mit Säuren oder quaternären Ammoniumsalzen vorliegen können.

**2.** Verbindungen der Formel (I), wie sie in Anspruch 1 definiert sind, dadurch gekennzeichnet, daß die Verbindung der Ringe in cis-Stellung ist, sowie ihre Additionssalze mit Säuren.

3. Verbindungen der Formel (I), wie sie in Anspruch 1 und 2 definiert sind, dadurch gekennzeichnet, daß A eine Gruppe $-CH_2-$ oder $-CH_2-O-$ bedeutet, sowie ihre Additionssalze mit Säuren.

4. Verbindungen der Formel (I), wie sie in den Ansprüchen 1 bis 3 definiert sind, dadurch gekennzeichnet, daß $R_1$ einen Methyl- oder Ethylrest bedeutet, $R_2$ ein Wasserstoffatom, einen Methyl- oder Ethylrest darstellt, Z einen Phenylrest bedeutet, gegebenenfalls substituiert durch einen oder zwei Reste, ausgewählt unter den Resten Methyl oder Ethyl, Methoxy oder Ethoxy, den Chlor- oder Bromatomen, den Trifluormethyl- oder Nitroresten, sowie ihre Additionssalze mit Säuren.

5. Verbindungen der Formel (I), wie sie in den Ansprüchen 1 bis 3 definiert sind, dadurch gekennzeichnet, daß $R_1$ einen Methyl- oder Ethylrest bedeutet, $R_2$ ein Wasserstoffatom, einen Methyl- oder Ethylrest darstellt, Z einen Naphthyl-, Pyridinyl-, Benzo[b]thienylrest bedeutet, sowie ihre Additionssalze mit Säuren.

6. (4aα,8α,8aα)-(±)-N-(Decahydro-1-methyl-8-chinolinyl)-3,4-dichlor-N-methylphenyl-acetamid und seine Additionssalze mit Säuren und (4aα,8α,8aα)-(±)-N-(Decahydro-1-methyl-8-chinolinyl)-2-(3,4-dichlorphenoxy)-N-methyl-acetamid und seine Additionssalze mit Säuren.

7. Verfahren zur Herstellung der Produkte der Formel (I), dadurch gekennzeichnet, daß man 8-Chlor-5,6,7, 8-tetrahydrochinolin der Einwirkung eines Produkts der Formel $X-R_1$ unterwirft, wobei $R_1$ einen Alkylrest mit 1 bis 5 Kohlenstoffatomen bedeutet und x ein Halogenatom darstellt, um ein Produkt der Formel (II)

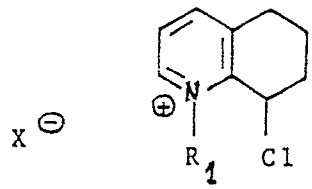

(II)

zu erhalten, das man mit einem Amin der Formel $NH_2-R_2$ umsetzt, wobei $R_2$ ein Wasserstoffatom oder ein Alkylrest mit 1 bis 5 Kohlenstoffatomen ist, um ein Produkt der Formel (III)

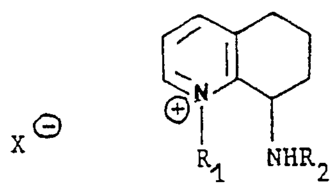

(III)

zu erhalten, das man reduziert, um ein Produkt der Formel (IV) in allen möglichen Enantiomeren- und Diastereoisomeren-Formen zu erhalten

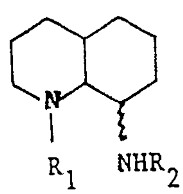

(IV)

das man mit einer Säure der Formel (V) oder einem funktionellen Derivat derselben

HOOC-(A)-Z (V)

kondensiert, wobei A und Z die vorstehend angegebenen Bedeutungen haben, um ein Produkt der

Formel (I) in allen möglichen enantiomeren und diastereoisomeren Formen zu erhalten, das aufgespalten werden kann, um die optisch aktiven Formen zu erhalten, und das man gewünschtenfalls mit einer Mineral- oder organischen Säure behandelt, um daraus das Salz herzustellen.

**8.** Als Arzneimittel die Produkte der Formel (I), wie sie in den Ansprüchen 1 bis 5 definiert sind.

**9.** Als Arzneimittel die Produkte der Formel (I), wie sie in Anspruch 6 definiert sind.

**10.** Pharmazeutische Zusammensetzungen, enthaltend als aktives Prinzip ein Arzneimittel gemäß den Ansprüchen 8 oder 9.

**11.** Als Zwischenprodukte die Produkte der Formel (III) und (IV).